# EUROPÄISCHE PATENTSCHRIFT

(11) **EP 1 099 676 B1**
(45) Veröffentlichungstag und Bekanntmachung des Hinweises auf die Patenterteilung: **29.10.2003**
(21) Anmeldenummer: 00118466.2
(22) Anmeldetag: 25.08.2000
(51) Int. Cl.: C07C 41/50

(54) **Verfahren zur Herstellung von 1,1,4,4-Tetramethoxybuten-2**
Process for the preparation of 1,1,4,4-tetramethoxybutene-2
Procédé de préparation du 1,1,4,4-tétraméthoxybutène-2

(30) Priorität: 30.09.1999 DE 19946816
(43) Veröffentlichungstag der Anmeldung: 16.05.2001
(73) Patentinhaber: BASF AKTIENGESELLSCHAFT, 67056 Ludwigshafen (DE)
(72) Erfinder: Wegner, Christoph, Dr., 67281 Kircheim (DE); Paust, Joachim, Dr., 67141 Neuhofen (DE); Ernst, Hansgeorg, Dr., 67346 Speyer (DE)

(56) Entgegenhaltungen:
- EP-A- 0 581 097
- US-A- 3 240 818
- SCHEEREN, J.W. ET AL.: "Chemistry of electron-rich conjugated polyenes (II). Synthesis of 1,1-dimethoxy and 1,1,4-trialkoxy-1,3-butadienes and of 1,1,6,6-tetramethoxy-1,3,5-hexatriene" RECUEIL. JOURNAL OF THE ROYAL NETHERLANDS CHEMICAL SOCIETY, Bd. 94, Nr. 8, August 1975 (1975-08), Seiten 196-198, XP001036956

## Beschreibung

Die Erfindung betrifft ein verbessertes Verfahren zur Herstellung von Tetramethoxybuten durch Umsetzung von 2,5-Dimethoxy-dihydrofuran mit Methanol in Gegenwart sauren organischen Ionenaustauschern.

Tetramethoxybuten ist ein wichtiges Zwischenprodukt zur Herstellung von C₁₀-Dialdehyd der Formel 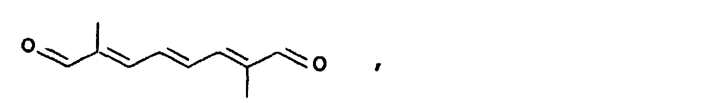 der seinerseits ein Schlüsselbaustein zur Synthese von Carotinoiden wie β-Carotin, Astaxanthin und Lycopin ist.

Nach dem von C.M. Cox, D.A. Whiting, J. Chem. Soc. Perkin Trans. 1 1991, 1907-1911, V.M. Likhosherstov, Russ. J. Org. Chem. 1983, 19, 1176-1178, sowie von S.M. Makin, N.I. Telefina, Zh. Obshch. Khim. 1962, 32, 1104-1109 und in US 2,768,976 und DE 956 946 beschriebenen Verfahren wird Tetramethoxybuten aus Furan und Brom in Methanol hergestellt. Nach 1,4-Bromaddition an Furan und anschließender nucleophiler Substitution des Broms durch Methanol entsteht hierbei Dimethoxydihydrofuran in situ. Durch die Bildung von Br bei der Brom-Substitution reagiert Dimethoxydihydrofuran direkt weiter zum Tetramethoxybuten.

Dieses Verfahren besitzt schwerwiegende Nachteile: Die Synthese muß bei Temperaturen von -30 bis -50°C durchgeführt werden; dies ist technisch nur schwer realisierbar: Die Verwendung von Brom erfordert einen hohen Sicherheitsaufwand. Darüber hinaus ist Brom ein teures Reagenz und hochkorrosiv und eine Ausrüstung der Produktionsanlage mit teuren Spezialmaterialien ist unumgänglich. Außerdem muß der äquimolar gebildete Bromwasserstoff bei der Aufarbeitung neutralisiert werden, wobei große Salzmengen als Abfall anfallen. Diese Synthese kann zwar prinzipiell auch mit preisgünstigerem Chlor durchgeführt werden, aber die für Brom aufgeführten Nachteile bleiben bestehen und die Reaktion läuft zudem deutlich langsamer ab.

Da Dimethoxydihydrofuran vorteilhaft durch Oxidation von Furan in Methanol gewonnen werden kann, ist auch schon dessen Umwandlung zu Tetramethoxybuten in Gegenwart einer starken gelösten Säure, z.B. von p-Toluolsulfonsäure oder Salzsäure beschrieben. Da es sich bei dieser Umsetzung nach der folgenden Reatkionsgleichung 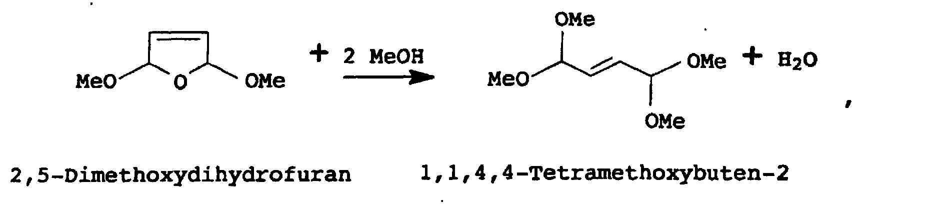 um eine spezielle Form einer Umacetalisierung unter Bildung eines Mols Wasser handelt, muß das freigesetzte Wasser dem Gleichgewicht entzogen werden. Nach dem Verfahren der EP-A 0 581 097 wird dieses Problem durch Zusatz von Trimethylorthoformiat gelöst, das aber relativ teuer ist. Zudem ist dieses Verfahren nicht einfach in den kontinuierlichen Maßstab übertragbar. Der gelöste Katalysator muß auch hier durch Basenzusatz neutralisiert werden, um die Reaktion am Optimum abbrechen zu können. Schließlich wurde gemäß der Veröffentlichung von N. Clauson-Kaas, J.T. Nielsen, E. Boss, Acta Chem. Scand. 1955, 9, 111-115, schon aprotische Lewis-Säuren, wie Bortrifluorid verwendet, doch betrug die Ausbeute an Tetramethoxybuten nur 9 % d.Th.

Ein weiteres Problem der vorbekannten Verfahren bestand darin, daß sich erhebliche Mengen des Nebenprodukts Pentamethoxybutan bilden, die die Ausbeute an Tetramethoxybuten mindern.

Es bestand daher die Aufgabe, ein Verfahren zu finden, das es erlaubt, in technisch einfacher Weise Tetramethoxybuten vor allem kontinuierlich in guter Ausbeute herzustellen und bei dem die Bildung von Pentamethoxybutan vermindert ist.

Diese Aufgabe wurde erfindungsgemäß dadurch gelöst, daß man die Umsetzung in Gegenwart von sauren organischen Ionenaustauschern und in Abwesenheit von Ameisensäure-ortho-alkylestern der Formel HC(OR)₃, in der R für -CH₃, -C₂H₅ oder -C₃H₇ steht, durchführt. Dabei erhält man vor allem bei einem Teilumsatz nicht nur hohe Selektivitäten, sondern auch geringere Mengen von Pentamethoxybutan als Nebenprodukt. Nach Destillation der Reaktionsprodukte unter Entfernung des gebildeten Wassers kann nicht umgesetztes Dimethoxybuten wieder in die Reaktion zurückgeführt werden.

Im einzelnen betrifft das neue Verfahren die Herstellung von 1,1,4,4-Tetramethoxybuten-2 durch Umsetzung von 2,5-Dimethoxydihydrofuran mit Methanol in Gegenwart von sauren organischen Ionenaustauschern und in Abwesenheit von Ameisensäure-ortho-alkylestern der Formel HC(OR)₃, in der R für -CH₃, -C₂H₅ oder -C₃H₇ steht.

Saure organische Ionenaustauscher sind an sich bekannte, teilweise vernetzte Kettenpolymere, insbesondere aus Styrol/Divinylbenzol, die saure Gruppen, vorzugsweise Sulfonsäuregruppen, enthalten, z.B. der Formel

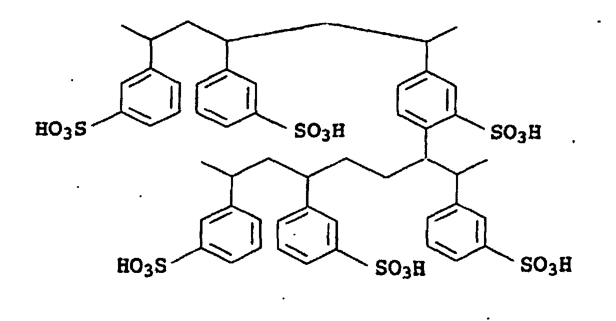

Ionenaustauscher dieser Art sind im Handel z.B. von DOW Chemical oder BAYER Aktiengesellschaft erhältlich.

Beispiele sind Dowex® 50WX, Serdolit Red®, Amberlyst® 15, Lewatit® K2431, Navion® H⁺, Amberlite® IR120, Duolite® C20, Lewatit® S100 und Lewatit® K2641.

Die Umsetzung erfolgt entweder ansatzweise z.B. in Suspensionsfahrweise oder vorzugsweise über einen fest angeordneten Katalysator, in einem kontinuierlich durchströmten Reaktor.

Bei der ansatzweisen Umsetzung wendet man in der Regel den Katalysator in Mengen von 1 bis 50, vorzugsweise 5 bis 30 Gew.-%, bezogen auf Dimethoxydihydrofuran, an. Methanol wird im allgemeinen im Überschuß über die stöchiometrisch erforderliche Menge, z.B. der 2- bis 40-fachen molaren Menge, vorzugsweise im 1,2- bis 20-fachen, insbesondere 1,5- bis 10-fachen molaren Überschuß über die stöchiometrische Menge hinaus eingesetzt.

Bevorzugt ist allerdings die kontinuierliche Arbeitsweise, bei der nur ein geringer Methanolüberschuß benötigt wird, ohne daß die Ausbeute erheblich geringer wird. Deshalb kann die Umsetzung mit einem Molverhältnis Dimethoxydihydrofuran zu Methanol von 1:2 bis 1:4, vorzugsweise von 1:2,4 bis 1:4 durchgeführt werden.

Die erfindungsgemäße Umsetzung findet in der Regel bei Temperaturen von -10 bis 100, vorzugsweise von 0 bis 40 und insbesondere bei 15 bis 30°C und Verweilzeiten von 1 bis 30 min, vorzugsweise 10 bis 60 min. statt.

In der ansatzweisen Ausführungsform der Erfindung wird Dimethoxydihydrofuran zusammen mit einem Überschuß Methanol z.B. mit einem sauren Ionenaustauscher bei Temperaturen von z.B. 0 bis 25°C mehrere Stunden gerührt, die Reaktionsmischung vom Katalysator abfiltriert und durch Destillation aufgearbeitet. Nichtumgesetztes Dimethoxydihydrofuran kann erneut eingesetzt werden.

Vorteilhaft führt man aber die Umsetzung kontinuierlich durch, indem Dimethoxydihydrofuran mit Methanol über einen fest angeordneten säuren Katalysator geleitet werden. Dabei wird die Reaktion zweckmäßig nur bis zu einem Teilumsatz z.B. weniger als 80 % der Theorie geführt. Die den Reaktor verlassende Mischung wird durch Destillation aufgearbeitet und das nicht umgesetzte Dimethoxydihydrofuran und das entwässerte Methanol in die Reaktion zurückgeführt.

Als Reaktor verwendet man bevorzugt Rohrreaktoren, in denen der saure Katalysator in einem oder mehreren Betten angeordnet ist.

### Beispiele

### (DMD = 2,5-Dimethoxydihydrofuran; TMB = 1,1,4,4-Tetramethoxybuten-2; PMB = 1,1,2,4,4-Pentamethoxybutan)

### Beispiel 1

Zu einer langsam gerührten Suspension eines sauren Ionenaustauschers (29 g Dowex® 50WX4; DOW Corp.) in Methanol (290 ml) wurde bei 0°C Dimethoxydihydrofuran (DMD 25,2 g, 0,19 mol) gegeben. Nach 14 h Rühren bei 0°C wurde vom Ionenaustauscher abfiltriert. Die GC-Analyse der methanolischen Lösung ergab 55,7 % Tetramethoxybuten (TMB), 42,0 % DMD und 2,0 % des Nebenprodukts Pentamethoxybutan (PMB). Dies entspricht einem Umsatz von 58 % und einer Selektivität von 97 %. Die Durchführung dieser Reaktion bei 10°C ergab nach 4 h Reaktionszeit lt. GC 57,3 % TMB, 39,4 % DMD und 2,9 % PMB. Dies entspricht einem Umsatz von 61 % und einer Selektivität von 95 %.

### Beispiel 2

Zu einer langsam gerührten Suspension eines sauren Ionenaustauschers (29 g Dowex® 50WX4 (DOW Corp.) in Methanol (290 ml) wurde bei 10°C DMD (25,2 g, 0,19 mol) gegeben. Nach 5 h Rühren bei 10°C wurde vom Ionenaustauscher abfiltriert. Die GC-Analyse der methanolischen Lösung ergab 59,1 % TMB, 36,4 % DMD und 4,0 % des Nebenprodukts PMB. Dies entspricht einem Umsatz von 64 % und einer Selektivität von 94 %.

### Beispiel 3

Zu einer langsam gerührten Suspension eines sauren Ionenaustauschers (10 g Lewatit K2641) in Methanol (100 ml) wurde bei 25°C DMD (8,70 g, 0,07 mol) gegeben. Nach 2 h Rühren bei 25°C wurde vom Ionenaustauscher abfiltriert. Die GC-Analyse der methanolischen Lösung ergab 53,2 % TMB, 41,2 % DMD und 4,4 % des Nebenprodukts PMB. Dies entspricht einem Umsatz von 59 % und einer Selektivität von 92 %.

### Beispiel 4

Zu einer langsam gerührten Suspension eines sauren Ionenaustauschers (10 g Lewatit K2641) in Methanol (100 ml) wurde bei 25°C DMD (2,72 g, 0,02 mol) gegeben. Nach 30 min. Rühren bei 25°C wurde vom Ionenaustauscher abfiltriert. Die GC-Analyse der methanolischen Lösung ergab 27,9 % TMB, 67,4 % DMD und 3,3 % des Nebenprodukts PMB. Dies entspricht einem Umsatz von 72 % und einer Selektivität von 95 %.

### Beispiel 5

Zu einer langsam gerührten Suspension eines sauren Ionenaustauschers (10 g Lewatit K2641, Bayer) in Methanol (100 ml) wurde bei 25°C Dimethoxydihydrofuran (DMD, 0,68 g, 0,005 mol) gegeben. Nach 15 min. Rühren bei 25°C wurde vom Ionenaustauscher abfiltriert. Die GC-Analyse der methanolischen Lösung ergab 34,6 % TMB, 63,1 % DMD und 1,5 % des Nebenprodukts PMB. Dies entspricht einem Umsatz von 65 % und einer Selektivität von 98 %.

### Beispiel 6

Eine Lösung von Dimethoxydihydrofuran (110 g) in Methanol (1000 g) wurde auf 5°C temperiert und von unten in eine senkrecht angebrachte Glassäule, gefüllt mit Ionenaustauscher Lewatit K2641, gepumpt. Die Pumpgeschwindigkeit wurde so gewählt, daß sich eine Kontaktzeit von 10 h ergab. Die Glassäule wurde ebenfalls auf 0°C temperiert. Die oben aus der Glassäule ablaufende methanolische Lösung wurde per GC analysiert: 54,8 % TMB, 41,1 % DMD, 3,2 % PMB. Dies entspricht einem Umsatz von 59 % und einer Selektivität von 94 %. Bei einer Verweilzeit von 15 h erhielt man 58,8 % TMB, 34,5 % DMD, 5,6 % PMB, entsprechend 66 % Umsatz und 91 % Selektivität.

### Beispiel 7

Eine Lösung von Dimethoxydihydrofuran (110 g) in Methanol (1000 g) wurde auf 0°C temperiert und von unten in eine senkrecht angebracht Glassäule, gefüllt mit Ionenaustauscher Dowex® 50WX4, gepumpt. Die Pumpgeschwindigkeit wurde so gewählt, daß sich eine Kontaktzeit von 3 h ergab. Die Glassäule wurde ebenfalls auf 0°C temperiert. Die oben aus der Glassäule ablaufende methanolische Lösung wurde per GC analysiert: 54,9 % TMB, 40,9 % DMD, 3,7 % PMB. Dies entspricht einem Umsatz von 60 % und einer Selektivität von 94 %.

### Beispiel 8

### Vergleich: Umsetzung mit p-Toluolsulfonsäure als Katalysator

Zu einer Lösung von Dimethoxydihydrofuran (37,0 g, 0,29 mol) in Methanol (420 ml) wurde bei 25°C p-Toluolsulfonsäure (1,9 g, 0,01 mol) zugefügt und die Mischung 24 h gerührt. Die GC-Analyse ergab folgende Werte: 53,0 % TMB, 40,0 % DMD, 7,0 % PMB. Dies entspricht einem Umsatz von 60 % und einer Selektivität von 88 %.

## Patentansprüche

1. Verfahren zur Herstellung von 1,1,4,4-Tetramethoxybuten-2 durch Umsetzung von 2,5-Dimethoxy-dihydrofuran mit Methanol in Gegenwart von Säuren, **dadurch gekennzeichnet, dass** man die Umsetzung in Gegenwart von sauren organischen Ionenaustauschern und in Abwesenheit von Ameisensäure-ortho-alkylestern der Formel HC(OR)₃, in der R für -CH₃, -C₂H₅ oder -C₃H₇ steht, durchführt.

2. Verfahren gemäß Anspruch 1, **dadurch gekennzeichnet, daß** man Sulfonsäuregruppen aufweisende Ionenaustauscher verwendet.

3. Verfahren gemäß Anspruch 1, **dadurch gekennzeichnet, daß** man das 2,5-Dimethoxy-dihydrofuran mit der 2 bis 40 molaren Menge Methanol bei Temperaturen von -10 bis 100°C kontinuierlich umsetzt.

4. Verfahren gemäß Anspruch 3, **dadurch gekennzeichnet, daß** man die Umsetzung bis zu einem Teilumsatz von weniger als 80 % der Theorie führt, in einer nachgeschalteten Destillation überschüssiges Methanol und gebildetes Wasser entfernt und nicht umgesetztes Dimethoxydihydrofuran in die Umsetzung zurückführt.

## Claims

1. A process for the preparation of 1,1,4,4-tetra-methoxy-2-butene by reacting 2,5-dimethoxydihydrofuran with methanol in the presence of acids, which comprises carrying out the reaction in the presence of acidic organic ion exchangers and in the absence of alkyl orthoformates of the formula HC(OR)₃ in which R is -CH₃, -C₂H₅ or -C₃H₇.

2. A process as claimed in claim 1, wherein ion exchangers having sulfonic acid groups are used.

3. A process as claimed in claim 1, wherein 2,5-dimethoxydihydrofuran is continuously reacted with 2 to 40 times the molar amount of methanol at from -10 to 100°C.

4. A process as claimed in claim 3, wherein the reaction is carried out to a partial conversion of less than 80% of theory, excess methanol and the water which has formed are removed in a subsequent distillation, and unconverted dimethoxy-dihydrofuran is returned to the reaction.

## Revendications

1. Procédé pour la préparation de 1,1,4,4-tétraméthoxybutène-2 par réaction de 2,5-diméthoxy-dihydrofuranne avec du méthanol en présence d'acides, **caractérisé par le fait qu'**on effectue la réaction en présence d'échangeurs d'ions organiques acides et en l'absence d'esters ortho-alkyliques d'acide formique de formule HC(OR)₃, dans laquelle R désigne -CH₃, -C₂H₅ ou -C₃H₇.

2. Procédé selon la revendication 1, **caractérisé par le fait qu'**on utilise des échangeurs d'ions présentant des groupes acide sulfonique.

3. Procédé selon la revendication 1, **caractérisé par le fait qu'**on fait réagir le 2,5-diméthoxy-dihydrofuranne en continu avec la quantité 2 à 40 fois molaire de méthanol à des températures de -10 à 100°C.

4. Procédé selon la revendication 3, **caractérisé par le fait qu'**on conduit la réaction jusqu'à une conversion partielle de moins de 80 % de la théorie, on élimine dans une distillation subséquente le méthanol en excès et l'eau formée, et on recycle dans la réaction le diméthoxydihydrofuranne n'ayant pas réagi.
